(19) Europäisches Patentamt
European Patent Office
Office européen des brevets

(11) **EP 2 544 585 B1**

(12) **EUROPEAN PATENT SPECIFICATION**

(45) Date of publication and mention of the grant of the patent:
**08.01.2014 Bulletin 2014/02**

(51) Int Cl.:
*A61B 5/02* (2006.01)    *A61B 5/027* (2006.01)
*A61B 5/0215* (2006.01)

(21) Application number: **11713017.9**

(22) Date of filing: **10.03.2011**

(86) International application number:
**PCT/GB2011/000344**

(87) International publication number:
**WO 2011/110817 (15.09.2011 Gazette 2011/37)**

(54) **METHOD AND APPARATUS FOR THE MEASUREMENT OF A FLUID FLOW RESTRICTION IN A VESSEL**

VERFAHREN UND VORRICHTUNG ZUR MESSUNG EINER FLUIDSTRÖMUNGSEINSCHRÄNKUNG IN EINEM GEFÄSS

MÉTHODE ET DISPOSITIF POUR LA MESURE D'UNE RESTRICTION DE FLUX DANS UN VAISSEAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2010 GB 201003964**

(43) Date of publication of application:
**16.01.2013 Bulletin 2013/03**

(73) Proprietor: **Imperial Innovations Limited London SW7 2PG (GB)**

(72) Inventors:
• **DAVIES, Justin**
  **London W2 1LA (GB)**
• **MAYET, Jamil**
  **London W2 1LA (GB)**

(74) Representative: **Potter Clarkson LLP**
**The Belgrave Centre**
**Talbot Street**
**Nottingham**
**NG1 5GG (GB)**

(56) References cited:
**WO-A2-01/21057      WO-A2-2006/037082**
**US-B1- 6 471 656**

• N. HADJILOIZOU ET AL: "Differences in cardiac microcirculatory wave patterns between the proximal left mainstem and proximal right coronary artery", AJP: HEART AND CIRCULATORY PHYSIOLOGY, vol. 295, no. 3, 1 January 2008 (2008-01-01), pages H1198-H1205, XP55006654, ISSN: 0363-6135, DOI: 10.1152/ajpheart.00510.2008
• J. E. DAVIES: "Evidence of a Dominant Backward-Propagating "Suction" Wave Responsible for Diastolic Coronary Filling in Humans, Attenuated in Left Ventricular Hypertrophy", CIRCULATION, vol. 113, no. 14, 11 April 2006 (2006-04-11), pages 1768-1778, XP55006653, ISSN: 0009-7322, DOI: 10.1161/CIRCULATIONAHA.105.603050 cited in the application
• J. E. DAVIES: "Use of simultaneous pressure and velocity measurements to estimate arterial wave speed at a single site in humans", AJP: HEART AND CIRCULATORY PHYSIOLOGY, vol. 290, no. 2, 1 January 2005 (2005-01-01), pages H878-H885, XP55006652, ISSN: 0363-6135, DOI: 10.1152/ajpheart.00751.2005 cited in the application
• PIJLS N H J ET AL: "CORONARY PRESSURE MEASUREMENT AND FRACTIONAL FLOW RESERVE", HEART, BMJ, LONDON, GB, vol. 80, 1 January 1998 (1998-01-01), pages 539-542, XP002937797, ISSN: 1355-6037

EP 2 544 585 B1

- **H. J. VERBERNE ET AL: "Effect of simultaneous intracoronary guidewires on the predictive accuracy of functional parameters of coronary lesion severity", AJP: HEART AND CIRCULATORY PHYSIOLOGY, vol. 292, no. 5, 1 January 2007 (2007-01-01), pages H2349-H2355, XP55006676, ISSN: 0363-6135, DOI: 10.1152/ajpheart.01042.2006**

**Description**

The present invention relates to methods and apparatus for determining the extent of a localised restriction to fluid flow in a vessel such as a pipe or tube. The invention has particular, though not exclusive, application in the measurement of a stenosis in a blood vessel, and is particularly useful in determining the magnitude of a coronary stenosis in the human or animal coronary system.

N. Hadjiloizou et al.: "Differences in cardiac microcirculatory wave patterns between the proximal left mainstem and proximal right coronary artery", Am. J. Physiol.: Heart Circ. Physiol., 295 (18 July 2008) pp. H1198-H1205, doi: 10.1152/ajpheart.00510.2008 relates to a study of the differences in cardiac microcirculatory wave patterns between the proximal left mainstem and proximal right coronary artery. Pressure, flow and electrocardiograms were acquired for 60 s. Pressure and flow velocity signals were ensemble averaged over the entire 60 s recording period. Equations that relate to changes in pressure and velocity are disclosed which provide pressure changes due to waves originating from proximal (aortic) and distal (microcirculatory) locations. An equation to calculate the local wave speed using simultaneous pressure and Doppler measurements is disclosed. Wave intensities are calculated using the local wave speed and respective pressure changes. Measurements are taken for both the left mainstem and the right coronary artery and the wave intensities compared.

Fractional flow reserve (FFR) is a technique widely applied in the coronary catheter laboratory in the assessment of coronary stenosis and the adequacy of stent deployment. FFR is defined as the pressure behind (or distal to) a stenosis relative to the pressure in front of (or proximal to) the stenosis. The result is a ratio, i.e. an absolute number. An FFR ratio of 0.5 indicates that a given stenosis results in a 50% drop in blood pressure across the stenosis. More generally, an FFR indicates the ratio of a maximal flow of fluid along a vessel in the presence of a restriction or constriction in the vessel compared with the maximal flow that would occur in the absence of that restriction or constriction.

The use of FFR has increased rapidly over the past few years as studies have demonstrated the limitations of visual assessment of stenoses and the harm which can arise from inappropriate angioplasty. It is conventionally performed by measuring the mean fall in pressure on either side of a coronary stenosis under conditions of maximal hyperaemia. However, under certain circumstances, e.g. following acute myocardial infarction, it can become unreliable. This could lead to inappropriate clinical decisions.

Whereas pressure in most vascular bed arises from a single input (i.e. the aortic end of the vessel), pressure in the coronary artery arises from both proximal (aortic end) and distal (microcirculatory end) contributions in approximately equal proportions. Distal pressure is determined by 2 factors:

(1) intrinsic (or 'passive') resistance via auto-regulation of the coronary microcirculation
(2) extrinsic (or 'active') resistance via compression of small microcirculatory vessels which run through the myocardium.

Current FFR assessment tries to reduce this distal pressure as much as possible by the administration of vasodilators such as adenosine to achieve 'maximal' hyperaemia. However, whilst the administration of vasodilators will reduce microcirculatory passive resistance, it cannot suppress the distal-originating microcirculatory pressure which arises from compression of the small vessels which run through the contracting myocardium.

Thus, a small inaccuracy in FFR is inherent as it is not possible to eliminate the active resistance component. Further, FFR can become more inaccurate under pathological processes when regulation of either intrinsic or extrinsic resistance is affected. Examples of passive resistance dysfunction include diabetes mellitus, acute coronary syndrome, post myocardial infarction and hibernating myocardium. Examples of active resistance dysfunction include when an artery subtends a hypokinetic or akinetic segment.

There is much published literature detailing such errors, which may explain why the close relationship between intravascular ultrasound (IVUS) and FFR in the highly regulated research laboratory is often not borne out in the clinical environment.

It is an object of the present invention to provide an improved and/or alternative method and apparatus for measuring the extent of a localised restriction to fluid flow in a vessel such as a pipe or tube. It is a further object of the invention to provide such a method and apparatus for use in the measurement of a stenosis in a blood vessel, and particularly though not exclusively in determining the magnitude or effects of a coronary stenosis in the human or animal coronary system.

According to one aspect, the present invention provides a method for determining a measure of constriction in a vessel conveying a fluid medium, the method comprising the steps of: a) taking a succession of first pressure measurements $P_1$ and a succession of corresponding first velocity measurements $U_1$ at a first location within the vessel, the first location being on a first side of a target region; b) taking a succession of second pressure measurements $P_2$ and a succession of corresponding second velocity measurements $U_2$ at a second location within the vessel, the second location being on a second side of the target region; c) for each location, determining wave speed c in the fluid medium as a function of the square of a change in pressure dP divided by the square of the corresponding change in velocity dU; d) for the first location, determining a forward pressure change $dP_1+$ as a function of the sum of the change in pressure $dP_1$ and the change in velocity $dU_1$; e) for the second location, determining a forward pressure change $dP_2+$ as a function of the sum of the change in pressure $dP_2$ and the change in velocity $dU_2$; f) determining a forward separated flow reserve indicative of the pressure drop across the target region as a function of the ratio of $dP_2+ / dP_1+$.

The first side of the target region may be upstream of the target region and the second side may be downstream of the target region. The wave speed may be determined at each location according to the equation $c = (1 / \rho) \sqrt{(\Sigma dP^2 / \Sigma dU^2)}$, where $\rho$ is the specific density of the fluid medium in the vessel. Steps d) and e) may comprise determining the forward pressure changes $dP_1+$ and $dP_2+$ according to the equations: $dP_1+ = (dP_1 + \rho c dU_1) / 2$ and $dP_2+ =.(dP_2 + \rho c dU_2) / 2$. Step f) may include integrating or summing multiple values of $dP_1+$ and $dP_2+$ to obtain forward pressure values $P_1+$ and $P_2+$ and determining the forward separated flow reserve as a function of the ratio $P_2+ / P_1+$. The method can be applied to a vessel in which there is a fluctuating pressure source on either side of the target region, such as in a vessel in the human or animal cardiac circulatory system. The succession of first and second pressure measurements and the succession of first and second velocity measurements may be taken over at least one complete cardiac cycle of the human or animal body. Corresponding pressure and velocity measurements may be taken simultaneously.

The present invention also provides an apparatus for determining a measure of constriction in a vessel conveying a fluid medium, the apparatus comprising: i) a pressure sensor and a velocity sensor for taking a succession of pressure and velocity measurements in the vessel at at least a first location upstream of a target region and a second location downstream of the target region; ii) a processing module adapted to: receive a succession of first pressure measurements $P_1$ and a succession of corresponding first velocity measurements $U_1$ taken at the first location within the vessel; receive a succession of second pressure measurements $P_2$ and a succession of corresponding second velocity measurements $U_2$ taken at the second location within the vessel; for each location, determine wave speed c in the fluid medium as a function of the square of a change in pressure dP divided by the square of the corresponding change in velocity dU; for the first location, determine a forward pressure change $dP_1+$ as a function of the sum of the change in pressure $dP_1$ and the change in velocity $dU_1$; and for the second location, determine a forward pressure change $dP_2+$ as a function of the sum of the change in pressure $dP_2$ and the change in velocity $dU_2$; and determine a forward separated flow reserve indicative of the pressure drop across the target region as a function of the ratio of $dP_2+ / dP_1+$.

The processing module may be adapted to determine the wave speed c at each location according to the equation $c = (1 / \rho) \sqrt{(\Sigma dP^2 / \Sigma dU^2)}$, where $\rho$ is the specific density of the fluid medium in the vessel. The processing module may be further adapted to determine said forward pressure changes $dP_1+$ and $dP_2+$ according to the equations: $dP_1+ =.(dP_1 + \rho c dU_1) / 2$ and $dP_2+ =.(dP_2 + \rho c dU_2) / 2$. The processing module may be further adapted to integrate or sum multiple values of $dP_1+$ and $dP_2+$ to obtain forward pressure values $P_1+$ and $P_2+$ and to determine the forward separated flow reserve as a function of the ratio $P_2+ / P_1+$. The apparatus may include means for monitoring a cardiac rhythm and for controlling said pressure sensor and said velocity sensor to collect said succession of pressure measurements and said succession of velocity measurements during a complete cardiac cycle.

Embodiments of the present invention will now be described by way of example and with reference to the accompanying drawings in which:

Figure 1 shows a schematic diagram of a vessel conveying a fluid medium in which the vessel has a constriction causing a pressure drop;

Figure 2 shows a flow diagram of a forward-pressure flow reserve measurement technique suitable for analysing a stenosis or other flow restriction in a vessel;

Figure 3 shows a schematic diagram of apparatus suitable for implementing the method of figure 2;

Figure 4 illustrates the differences in the ratio between proximal-originating waves and distal-originating waves in a normal ventricle and in a severely hypokinetic ventricle;

Figure 5 is a schematic illustration of the fall in fractional flow reserve with increasing coronary stenosis in normal and hypothesised impaired left ventricular function showing that in the normally contracting LV, fractional flow reserve falls with increasing coronary stenosis (solid line), whereas in the hypothesised impaired left ventricular model (dotted line), fractional flow reserve falls by a much reduced amount; and

Figure 6 shows a set of graphs illustrating the separation of the total measured pressure into its forward and backward-travelling components, as a function time.

Recently it has been described how it is possible to separate the aortic and microcirculatory components of a pressure wave within the coronary artery. See J E Davies et al: Evidence of a dominant backward-propagating "suction" wave responsible for diastolic coronary filling in humans, attenuated in left ventricular hypertrophy; Circulation 2006 April 11;113(14):1768-78 and J E Davies et al: Use of simultaneous pressure and velocity measurements to estimate arterial wave speed at a single site in humans; Am J Physiol Heart Circ Physiol 2006 February; 290(2):H878-H885.

The present inventors have recognised that it is possible to assess the severity of a stenosis, without the need to account for (or remove) the distal pressure component, by using a forward-pressure flow reserve technique described herein. Forward-pressure flow reserve overcomes limitations in conventional FFR by separating out the proximal and distal (or 'forward' and 'backward') components of the pressure waveform. The backward pressure component can be removed. Assessment of the coronary stenosis is simplified becoming very similar to assessment of aortic stenosis where there is a single pressure source (i.e. the left ventricle).

Coronary pressure separation has several advantages. Firstly, it does not require the administration of adenosine to vasodilate the coronary microcirculation. Secondly, it is likely to be independent of left ventricular function making it applicable in acute coronary syndromes, post myocardial infarction and hibernating myocardium, where conventional FFR is contra-indicated as an assessment technique.

In practical terms, separated pressure can be determined by measuring simultaneous pressure (P) and flow velocity (U), and calculating the pressure originating in the forward (e.g. aortic) end, $P_+$, (Equation 1) and the pressure originating from the backward (e.g. microcirculatory) end, $P_-$ (Equation 2).

$$P_+ = \Sigma\ (1/2)\,(dP + \rho cdU) \qquad\qquad \text{(Equation 1)}$$

$$P_- = \Sigma\ (1/2)\,(dP - \rho cdU) \qquad\qquad \text{(Equation 2)}$$

dP represents a measured pressure change; dU represents a measured velocity change; c is the wave speed; and $\rho$ is the density of the fluid medium, e.g. blood. The severity of coronary stenosis can be determined according to an equation that is similar to conventional FFR. In conventional FFR, the measure of stenosis (i.e. the FFR ratio defined above) is determined by:

$$\text{Conventional FFR} = (\text{distal pressure}) / (\text{proximal pressure}) \qquad \text{(Equation 3)}$$

The measure of FFR may alternatively be expressed in terms of the forward-pressure flow reserve, i.e. without the effects of the backward pressure originating from the microcirculatory end:

$$\text{Forward pressure flow reserve} = (\text{distal } P_+) / (\text{proximal } P_-) \qquad \text{(Equation 4)}$$

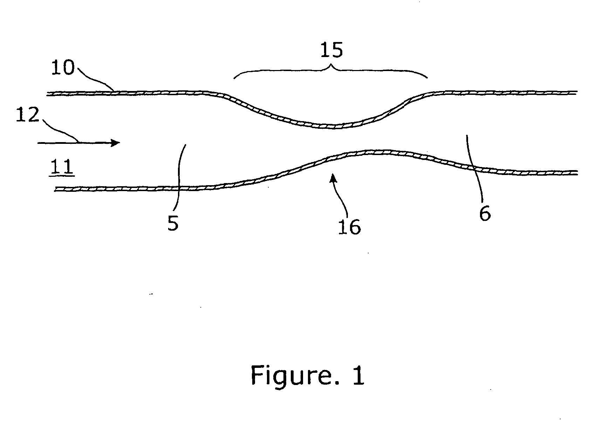

Figure 1 shows a schematic diagram of a vessel 10 for conveying a fluid medium 11 in an axial direction 12 along the vessel. The vessel 10 may be a pipe or tube and in one important context, may comprise a part of a vessel in the human or animal coronary system. A constriction 15 in the vessel 10 exemplifies a target region 16 for which it is desirable to make a measurement of the effect of that constriction on flow of fluid through the vessel. In one context, the constriction 15 may be a coronary artery stenosis and it is required to determine a measure of the drop in fluid pressure across the constriction to determine a ratio of the maximal flow through the vessel compared with the maximal flow that would occur without the constriction. Regions 5 and 6 represent first and second locations at which pressure and velocity measurements may be taken in accordance with the method to be described below. The first location 5 is on a first side of the target region 15 and the second location 6 is on a second side of the target region 15. The first location 5 could be at the proximal or aortic side of a coronary (or other) stenosis and the second location 6 would then be at the distal or microcirculatory side of the coronary (or other) stenosis. It is preferred that the distance of the first location (the proximal or aortic side) from the stenosis is at least 1.5 times the vessel diameter in the non-restricted part of the vessel.

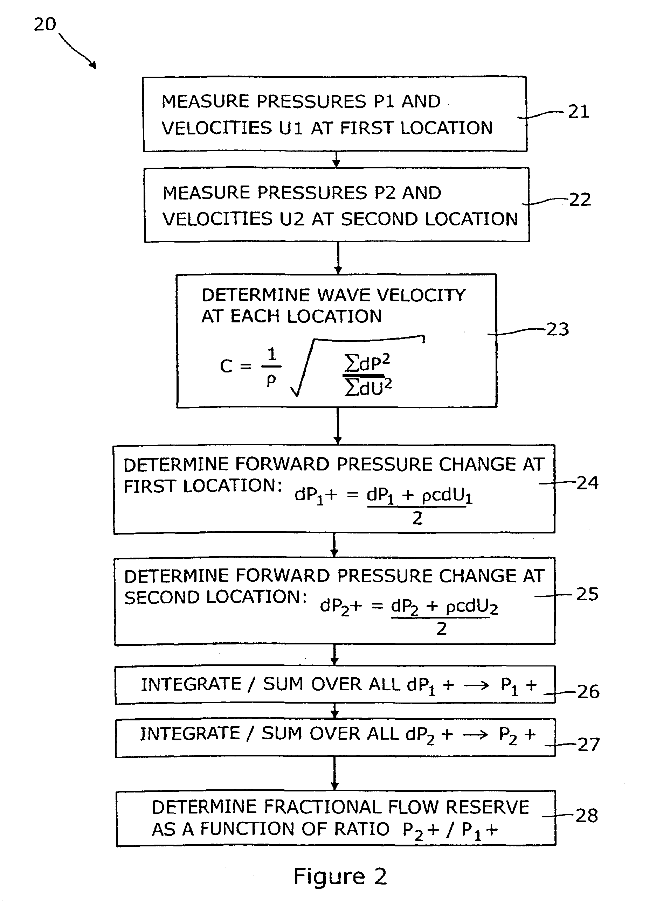

Figure 2 shows an exemplary method 20 for determining a measure of the constriction 15 in the vessel 10. A succession of first pressure measurements $P_1$ and a succession of corresponding first velocity measurements $U_1$ are taken at the first location 5 (step 21). A succession of second pressure measurements $P_2$ and a succession of corresponding second velocity measurements $U_2$ are taken at the second location 6 (step 22). Each pressure measurement and its corresponding velocity measurement are preferably taken substantially simultaneously.

The wave speed c at each of the first and second locations 5, 6 is determined as a function of the square of the change in pressure dP divided by the square of the corresponding change in velocity dU (step 23). The change in pressure dP is preferably determined from a pair of the succession of first pressure measurements $P_1$ and, correspond-

ingly, from a pair of the succession of second pressure measurements $P_2$. The change in velocity dU is preferably determined from a pair of the succession of first velocity measurements $U_1$ and, correspondingly, from a pair of the succession of second velocity measurements $U_2$. More preferably, a succession of pressure measurements and corresponding velocity measurements is taken over a period of time to generate multiple dP and dU measurements which can be aggregated to improve signal to noise ratio. The wave speed may be calculated for a succession of pairs of pressure and velocity measurements which are summed, and the square root of the sum taken. The wave speed can thereby be determined for a succession of measurement times, at each of the first and second locations 5, 6, according to the formula:

$$c = (1 / \rho) \; \sqrt{} \; (\Sigma dP^2 / \Sigma dU^2) \qquad\qquad \text{(Equation 5)}$$

where $\rho$ is the specific density of the fluid medium in the vessel. In a preferred context, the fluid medium is blood with a density of 1050 kg/m$^3$.

A forward pressure change dP+ is then determined as a function of the sum of the change in pressure dP and the corresponding (simultaneous) change in velocity dU. More preferably, the forward pressure change $dP_1+$ at the first location is determined according to the equation:

$$dP_1+ = (dP_1 + \rho c dU_1) / 2 \qquad\qquad \text{(Equation 6)}$$

and the forward pressure change $dP_2+$ at the second location is determined according to the equation:

$$dP_2+ = (dP_2 + \rho c dU_2) / 2 \qquad\qquad \text{(Equation 7)}$$

as shown in steps 24 and 25.

The values of $dP_1+$ are preferably summed or integrated over all successive measurements to obtain a forward pressure value $P_1+$ at the first location (step 26). The values of $dP_2+$ are also preferably summed or integrated over all successive measurements to obtain a forward pressure value $P_2+$ at the second location (step 27).

The flow reserve for the forward pressures is then determined as a function of the ratio $P_2+ / P_1+$ (or, if single pressure change measurements are used, $dP_2+ / dP_1+$). If the first location 5 comprises the proximal or aortic side of the stenosis and the second location 6 comprises the distal or microcirculatory side of the stenosis then the forward pressure flow reserve, $FPFR_{forward} = P+_{distal} / P+_{proximal}$. Thus, in a preferred arrangement, the first location 5 is upstream of the target region 15 and the second location 6 is downstream of the target region 15 (assuming continuous positive flow).

In the context of measurement of a coronary stenosis, preferably the successions of pressure and velocity measurements are made over at least one full cardiac cycle and preferably over a whole number of cardiac cycles. The mean and maximum values of $P_1+$ and $P_2+$ may be used in the calculation of FPFR to derive a value of $FPFR_{mean}$ and $FPFR_{max}$. The values of $dP_1+$ and $dP_2+$ used to obtain a forward pressure value $P_1+$ and a forward pressure value $P_2+$ may be taken from selected parts of one or more cardiac cycles or, as stated above, over one or more entire cardiac cycles. Preferably, at least five or ten measurements of $dP_1+$ and $dP_2+$ are used for each cardiac cycle..

*Apparatus*

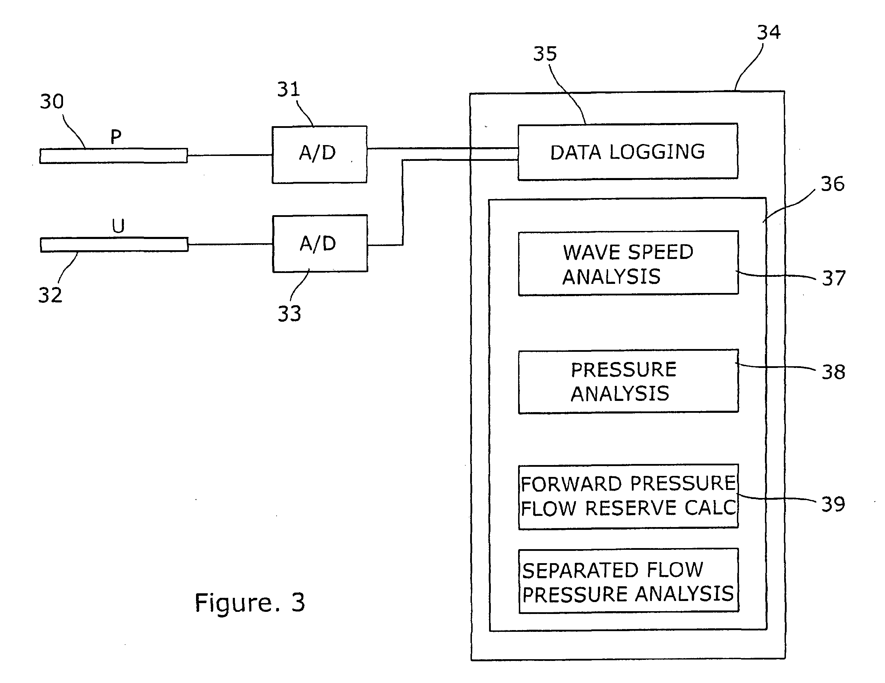

Apparatus suitable for carrying out the methods described above is shown generally in figure 3.

A pressure sensing device 30 is used to generate signals indicative of the instantaneous pressure at a selected location 5 or 6 in the vessel 10. These pressure signals are transmitted to a suitable analogue-to-digital converter 31 to generate a series of pressure measurements as a function of time taken at the selected location, e.g. the succession of first pressure measurements $P_1$ and the succession of second pressure measurements $P_2$. Similarly, a velocity sensing device 32 is used to generate signals indicative of the instantaneous fluid velocity at substantially the same selected location 5 or 6 as the pressure sensing device 30. These fluid velocity signals are transmitted to a suitable analogue-to-digital converter 33 to generate a series of fluid velocity measurements as a function of time taken at the selected location, e.g. the succession of first velocity measurements $U_1$ and the succession of second velocity measurements $U_2$. The corresponding pressure and velocity measurements are preferably taken at substantially the same times.

The pressure sensing device 30 may be any suitable transducer or other device capable of providing a direct or indirect measurement of pressure at a selected location in the vessel 10. The pressure sensing device may be an in-situ pressure transducer located within the fluid in the vessel 10 at the selected location 5, 6 or may be a remotely located active or passive sensor using any detectable radiation from the fluid flow or its confining vessel that can be used to determine pressure whether acoustic, electromagnetic, magnetic or otherwise. For example, in the coronary arteries and aorta, the PrimeWire™, FloWire™ and ComboWire™ XT by Volcano Corporation may be used as in-situ sensors.

The fluid velocity sensing device 32 similarly may be any suitable transducer or other device capable of providing a direct or indirect measurement of fluid velocity at a selected location in the vessel 10. The fluid velocity sensing device 32 may be an in-situ transducer located within the fluid in the vessel at the selected location 5, 6 or may be a remotely located active or passive sensor using any detectable radiation from the fluid flow that can be used to determine fluid velocity whether acoustic, electromagnetic, magnetic or otherwise, such as Doppler ultrasound techniques. In the coronary arteries and aorta, the WaveWire™, FloWire™ and ComboWire™ XT products referenced earlier may be used as in-situ sensors. The expression 'detectable radiation from the fluid flow' is intended to encompass any active or reflected radiation or re-radiation of energy from the fluid itself or from any agents or markers carried in the fluid.

The same pressure sensing device 30 may be used to obtain the succession of first pressure measurements $P_1$ at the first location 5 and the succession of second pressure measurements $P_2$ at the second location 6, at separate times. Similarly, the same velocity sensing device 32 may be used to obtain the succession of first velocity measurements $U_1$ at the first location 5 and the succession of second velocity measurements $U_2$ at the second location 6, at separate times. Alternatively, combined sensors, such as the ComboWire™ sensor, may be configured to take measurements at both the first and second locations simultaneously. The ComboWire™ sensor is a steerable guide wire with a pressure transducer mounted proximal to the tip and an ultrasound transducer mounted at the tip. It can be used to measure simultaneous pressure and blood flow velocity in blood vessels including both coronary and peripheral vessels.

The data streams from analogue-to-digital converters 31, 33 are passed to a data logging module 35, preferably implemented by a computer 34. The computer 34 includes a separated pressure flow reserve analysis module 36 for implementing the algorithms described herein.

A first processing module 37 (wave speed analysis module) determines the wave speeds at the first and second locations, preferably in accordance with the equation given for c above (equation 5). A second processing module 38 (pressure analysis module) determines the forward pressure changes at the first location, preferably in accordance with the expression for $dP_1+$ given above (equation 6). The second processing module 38 also determines the forward pressure changes at the second location, preferably in accordance with the expression for $dP_2+$ given above (equation 7). The wave speed c may be determined by sampling pressure and fluid velocity over one or more entire cardiac cycles at the selected location and averaging over those cycles.

The computer 34 preferably includes a further computation module 39 to integrate or sum the forward pressure changes at the first and second locations in accordance with steps 26 and 27 of figure 2, and to determine the forward pressure flow reserve $FPFR_{torward}$ preferably in accordance with step 28 of figure 2.

$FPFR_{forward}$ gives a measure of the severity of the coronary stenosis. The measurement produced in this manner, i.e. using only forward-travelling (aortic-originating) pressure waves, is substantially less influenced or not influenced by regional variation in myocardial contractility or autonomic dysregulation of the coronary microcirculation. The data processing of computer 34 may be performed on any suitable apparatus, such as a suitable programmed computer system or in a custom-built hardware / software measurement console of a pressure and flow velocity measurement. It will be understood that the distribution of the computational functions in hardware and software can be handled differently than the exemplary analysis modules as shown in figure 3 and can be implemented in any suitable combination of hardware and software.

A discussion of the clinical significance of use of separation of forward and backward separated pressures in the assessment of coronary stenosis is provided in Appendix 1.

While the techniques of the invention have been principally described in connection with analysis of stenoses or other constrictions in the coronary system, the techniques described may be applicable also to other systems such as the renal circulatory system or any other system where flow of fluid past a constriction is governed by forward and backward travelling pressure waves.

Other embodiments are intentionally within the scope of the accompanying claims.

APPENDIX 1

This new forward pressure flow reserve technique has several key therapeutic advantages over conventional FFR.

1. assessment of coronary stenosis immediately after acute myocardial infarction
2. assessment of coronary stenosis within 5 days of acute coronary syndrome
3. assessment of coronary stenosis in patients with regional wall motion abnormalities

4. assessment of coronary stenosis in subjects with microcirculatory disease

5. relinquished need for administration of adenosine.

These benefits may significantly increase the number of patients suitable for FFR-type assessment and have a positive impact on the total numbers of coronary revascularisation cases performed. Currently in the UK, approximately 30% of the case load is from acute admissions in patients with acute myocardial infarction or acute coronary syndrome. In this population, FFR is contra-indicated and has been found to be inconsistent at best and frequently unreliable.

Forward-pressure flow reserve overcomes or mitigates these limitations by separating out the proximal and distal components of the pressure waveform. As forward-pressure flow reserve is able to remove the backward pressure from the forward pressure component it negates the need to administer powerful vasodilators such as adenosine.

This has several specific advantages.

1. Overcomes limitations of adenosine intolerance (asthma, COPD etc.)

2. Overcomes limitations of adenosine resistance

3. Avoids insertion of secondary central venous sheath

4. Reduces overall time of case.

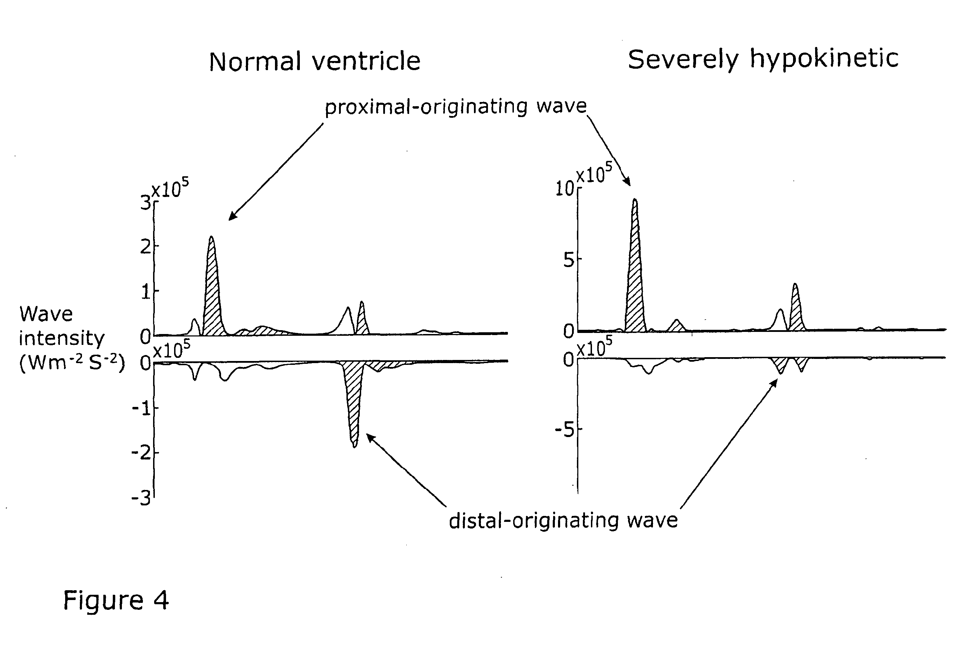

From pilot data, the inventors have found large differences in the ratio between proximal- and distal-originating waves in the normal and severely hypokinetic ventricle. In some cases, a greater than 80% reduction is observed in the proximal / distal ratio in the artery subtending severely hypokinetic territory when compared to an artery subtending myocardium contracting normally. This is shown in figure 4. Pressure and flow velocity were recorded using intra-arterial wires in the left anterior descending artery and wave intensity calculated for each artery. In the ventricle with preserved function, the proximal / distal ratio was approximately 1, whereas in the artery subtending a segment of severely hypokinetic function this ratio is markedly increased. This suggests regional myocardial function differentially affects pressure originating from proximal and distal origins.

Fractional Flow Reserve (FFR) assumes that coronary pressure originates solely from the proximal (aortic) end of the artery, and that the forces exerted on the intramural coronary vessels by the transmitted cavity pressure and contracting myocardium do not contribute to coronary artery pressure. The inventors have demonstrated that this is not the case, but instead that coronary pressure is composed of approximately 50% forward-travelling (aortic-originating) and 50% backward-travelling pressure components (see figure 4, left-hand graph). It is believed that in subjects with regional variation in myocardial contractility: (i) backward-travelling pressure is markedly reduced (as shown in the right hand graph of figure 4), and (ii) that it is not possible to determine whether a fall in FFR is due to a haemodynamically significant coronary stenosis or regional variation in myocardial contractility. Using separated-pressure components as described in this patent application allows the haemodynamic significance of coronary stenosis to be quantified independently of regional variation in myocardial contractility. This technique can be adopted routinely in clinical practice, and negates the need for routine administration of intravenous adenosine.

Fractional flow reserve (FFR) is increasingly used in the assessment of the physiological significance of a coronary stenosis[1,2,3,4] and adequacy of stent deployment[5] in the cardiac catheter laboratory. This technique is based on simple premise that the greater the stenosis the greater the fall in pressure between the aorta and downstream of the stenosis.

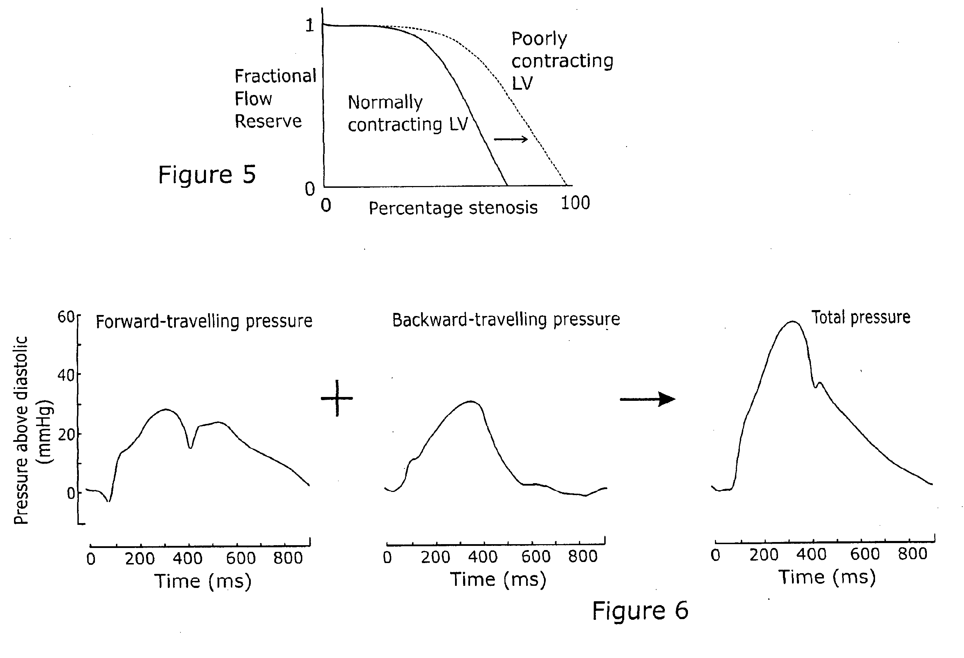

FFR is based on the assumption that pressure changes arise purely from the aortic end of the coronary artery. However, several studies including our own have clearly demonstrated that coronary artery pressure is influenced by changes in pressure from both ends of the vessel[6-9]. Probably the most widely accepted models of coronary artery flow are the intra-myocardial pump[6] and time-varying elastance models[9]. Both predict the existence of retrograde coronary blood flow during systole as a result of raised intramyocardial pressure and myocardial contraction causing compression of the small microcirculatory vessels[6]. Such retrograde flow has been confirmed by measurements in dogs in vivo using needle probe videomicroscopy[10] and Doppler flow probe[11]. This indicates the presence of a considerable backward-directed pressure gradient in systole Our work[8] has characterised this backward pressure gradient as a backward-travelling wave seen in the coronary arteries of humans in early systole due to compression of the coronary microvasculature. In diastole there is a corresponding 'suction' of blood into the coronary microvasculature as a result of the decompression of intramural vessels accompanying myocardial relaxation. We have recently demonstrated that this backward suction wave is depressed in left ventricular hypertrophy[8]. Therefore there may be a smaller backward-travelling pressure component in other circumstances which impair the lusitropic behaviour of the region of myocardium supplied by a particular coronary artery. The existence of pressure gradients generated by the contracting and relaxing myocardium will affect the measurement of FFR significantly, perhaps by 50% or more (Figure 5).

The limitations of the assumption of a unidirectional pressure gradient implicit in calculation of FFR (i.e. that there is no significant backward-travelling contribution to coronary pressure) are recognised as a potential source of error in microvascular disease and left ventricular dysfunction[2,12,13]. However, until recently, there has been no means of dealing with it, i.e. the absence of a technique to separate the coronary pressure waveform into its forward- and backward-travelling components in human subjects.

We have recently developed a new technique (the single-point technique) which, in combination with wave intensity analysis, enables coronary pressure to be separated into its forward and backward components on the basis of simultaneous recordings of pressure and flow velocity[14] (Figure 6). Such measurements are now quite feasible using a commercially available combined pressure-flow wire designed for intra-coronary use (Combiwire, Volcano), and negate the need for administration of adenosine. Using this technique in in-vivo human studies, we have identified the waves responsible for coronary blood flow, and separated the coronary pressure waveform into its forward-(aortic originating) and backward-travelling (microcirculatory originating) components[8].

These studies have demonstrated the backward-travelling pressure component in human coronaries is of the same magnitude as the large forward-travelling pressure component. Since coronary pressure is just as much a result of backward-travelling pressure compenents[14] as it is of forward-travelling components, if we are interested in the forward propagation of pressure it would be more appropriate to measure FFR using the isolated forward-travelling pressure component. By thus removing the backward-travelling pressure component, we can eliminate the influence of regional variation of left ventricular function, microvascular dysfunction and right atrial pressure allowing a more accurate assessment of the haemodynamic significance of a stenosis.

Figure 6 shows a set of graphs illustrating the separation of the total measured pressure into its forward and backward-travelling components as a function time. Simultaneous pressure and flow velocity were measured in the circumflex artery of a 47 year old man. Wave intensity analysis was applied to separate coronary pressure into its forward and backward-travelling components. Forward pressure appears different from aortic pressure due to the large impedance mismatch between the coronary artery and the aorta. At such sites the backward-travelling wave is re-reflected back into the coronary as an expansion (or suction) wave which reduces pressure.

Reference List

(1) Dawkins KD, Gershlick T, de BM et al. Percutaneous coronary intervention: recommendations for good practice and training. Heart 2005 December; 91 Suppl 6:vi1-27.

(2) Blows LJ, Redwood SR. The pressure wire in practice. Heart 2007 April; 93(4):419-22.

(3) Pijls NH, van Son JA, Kirkeeide RL, de BB, Gould KL. Experimental basis of determining maximum coronary, myocardial, and collateral blood flow by pressure measurements for assessing functional stenosis severity before and after percutaneous transluminal coronary angioplasty. Circulation 1993 April; 87(4):1354-67.

(4) Pijls NH, de BB, Peels K et al. Measurement of fractional flow reserve to assess the functional severity of coronary-artery stenoses. N Engl J Med 1996 June 27;334(26):1703-8.

(5) Pijls NH, Klauss V, Siebert U et al. Coronary pressure measurement after stenting predicts adverse events at follow-up: a multicenter registry. Circulation 2002 June 25; 105(25):2950-4.

(6) Spaan JA, Breuls NP, Laird JD. Diastolic-systolic coronary flow differences are caused by intramyocardial pump action in the anesthetized dog. Circ Res 1981 September; 49(3):584-93.

(7) Gregg DE, Sabiston DC. Effect of cardiac contraction on coronary blood flow. Circulation 1957 January; 15(1): 14-20.

(8) Davies JE, Whinnett ZI, Francis DP et al. Evidence of a dominant backward-propagating "suction" wave responsible for diastolic coronary filling in humans, attenuated in left ventricular hypertrophy. Circulation 2006 April 11; 113 (14):1768-78.

(9) Krams R, Sipkema P, Westerhof N. Varying elastance concept may explain coronary systolic flow impediment. Am J Physiol 1989 November; 257(5 Pt 2):H1471-H1479.

(10) Hiramatsu O, Goto M, Yada T et al. In vivo observations of the intramural arterioles and venules in beating canine hearts. J Physiol 1998 June 1; 509 (Pt 2):619-28.

(11) Chilian WM, Marcus ML. Phasic coronary blood flow velocity in intramural and epicardial coronary arteries. Circ Res 1982 June; 50(6):775-81.

(12) Siebes M, Chamuleau SA, Meuwissen M, Piek JJ, Spaan JA. Influence of hemodynamic conditions on fractional flow reserve: parametric analysis of underlying model. Am J Physiol Heart Circ Physiol 2002 October; 283(4):H1462-H1470.

(13) Coronary flow is not that simple! Spaan JA. Heart. 2009 May; 95(9):761-2

(14) Davies JE, Hadjiloizou N, Francis DP, Hughes AD, Parker KH, Mayet J. The role of the coronary microcirculation in determining blood flow. Artery Research 1[S1], S31-S32. 2006. Ref Type: Abstract

(15) Kim RJ, Wu E, Rafael A et al. The use of contrast-enhanced magnetic resonance imaging to identify reversible myocardial dysfunction. N Engl J Med 2000 November 16;343(20):1445-53.

(16) Perera D, Biggart S, Postema P et al. Right atrial pressure: can it be ignored when calculating fractional flow reserve and collateral flow index? J Am Coll Cardiol 2004 November 16; 44(10):2089-91.

(17) Davies JE, Whinnett ZI, Francis DP et al. Use of simultaneous pressure and velocity measurements to estimate arterial wave speed at a single site in humans. Am J Physiol Heart Circ Physiol 2006 February; 290(2):H878-H885.

(18) Parker KH, Jones CJ, Dawson JR, Gibson DG. What stops the flow of blood from the heart? Heart Vessels 1988; 4(4):241-5.

(19) Davies JE, Parker KH, Francis DP, Hughes AD, Mayet J. What is the role of the aorta in directing coronary blood flow? Heart 2008 December; 94(12):1545-7.

(20) Hadjiloizou N, Davies JE, Malik IS et al. Differences in cardiac microcirculatory wave patterns between the proximal left mainstem and proximal right coronary artery. Am J Physiol Heart Circ Physiol 2008 September; 295 (3):H1198-H1205.

**Claims**

1. A method (20) of determining a measure of constriction (15) in a vessel (10) conveying a fluid medium (11), the method comprising the steps of:

   a) taking (21) a succession of first pressure measurements $P_1$ and a succession of corresponding first velocity measurements $U_1$ at a first location (5) within the vessel (10), the first location (5) being on a first side of a target region (16);
   b) taking (22) a succession of second pressure measurements $P_2$ and a succession of corresponding second velocity measurements $U_2$ at a second location (6) within the vessel (10), the second location (6) being on a second side of the target region (16);
   c) for each location, determining (23) wave speed c in the fluid medium (11) as a function of the square of a change in pressure dP divided by the square of the corresponding change in velocity dU;
   d) for the first location (5), determining (24) a forward pressure change $dP_1+$ as a function of the sum of the change in pressure $dP_1$ and the change in velocity $dU_1$;
   e) for the second location (6), determining (25) a forward pressure change $dP_2+$ as a function of the sum of the change in pressure $dP_2$ and the change in velocity $dU_2$;
   f) determining (28) a forward separated flow reserve indicative of the pressure drop across the target region (16) as a function of the ratio of $dP_2+$ / $dP_1+$.

2. The method of claim 1 in which the first side of the target region (16) is upstream of the target region and the second side is downstream of the target region.

3. The method of claim 1 in which step c) comprises determining (23) the wave speed c at each location according to the equation $c = (1 / \rho) \sqrt{(\Sigma dP^2 / \Sigma dU^2)}$, where $\rho$ is the specific density of the fluid medium (11) in the vessel (10).

4. The method of claim 1 in which steps d) and e) comprise determining (24, 25) said forward pressure changes $dP_1+$ and $dP_2+$ according to the equations: $dP_1+ = (dP_1 + \rho c dU_1) / 2$ and $dP_2+ = (dP_2 + \rho c dU_2) / 2$.

5. The method of claim 1 in which step f) includes integrating (26, 27) or summing multiple values of $dP_1+$ and $dP_2+$ to obtain forward pressure values $P_1+$ and $P_2+$ and determining (28) the forward separated flow reserve as a function of the ratio $P_2+$ / $P_1+$.

6. The method of claim 1 applied to a vessel (10) in which there is a fluctuating pressure source on either side of the target region (16).

7. The method of claim 6 applied to a vessel (10) in the human or animal cardiac circulatory system.

8. The method of claim 7 in which the succession of first and second pressure measurements and the succession of first and second velocity measurements are taken (21,22) over at least one complete cardiac cycle.

9. The method of claim 1 in which corresponding pressure and velocity measurements are taken simultaneously.

10. Apparatus for determining a measure of constriction (15) in a vessel (10) conveying a fluid medium (11), the apparatus comprising:

a pressure sensor (30) and a velocity sensor (32) for taking (21, 22) a succession of pressure and velocity measurements in the vessel (10) at at least a first location (5) upstream of a target region (16) and a second location (6) downstream of the target region (16);

a processing module (35, 36) adapted to:

receive a succession of first pressure measurements $P_1$ and a succession of corresponding first velocity measurements $U_1$ taken at the first location (5) within the vessel (10);

receive a succession of second pressure measurements $P_2$ and a succession of corresponding second velocity measurements $U_2$ taken at the second location (6) within the vessel (10);

for each location, determine wave speed c (23) in the fluid medium (11) as a function of the square of a change in pressure dP divided by the square of the corresponding change in velocity dU;

for the first location (5), determine (24) a forward pressure change $dP_1+$ as a function of the sum of the change in pressure $dP_1$ and the change in velocity $dU_1$; and

for the second location (6), determine (25) a forward pressure change $dP_2+$ as a function of the sum of the change in pressure $dP_2$ and the change in velocity $dU_2$; and

determine (28) a forward separated flow reserve indicative of the pressure drop across the target region (16) as a function of the ratio of $dP_2+ / dP_1+$.

11. The apparatus of claim 10 in which the processing module (36) is further adapted to determine (23) the wave speed c at each location according to the equation $c = (1 / \rho) \sqrt{(\Sigma dP^2 / \Sigma dU^2)}$, where $\rho$ is the specific density of the fluid medium (11) in the vessel (10).

12. The apparatus of claim 10 in which the processing module (36) is further adapted to determine (24, 25) said forward pressure changes $dP_1+$ and $dP_2+$ according to the equations: $dP_1+ = (dP_1 + pcdU_1) / 2$ and $dP_2+ = (dP_2 + pcdU_2) / 2$.

13. The apparatus of claim 10 in which the processing module (36) is further adapted to integrate (26, 27) or sum multiple values of $dP_1+$ and $dP_2+$ to obtain forward pressure values $P_1+$ and $P_2+$ and to determine (28) the forward separated flow reserve as a function of the ratio $P_2+ / P_1+$.

14. The apparatus of claim 10 further including means for monitoring a cardiac rhythm and for controlling said pressure sensor and said velocity sensor to collect said succession of pressure measurements and said succession of velocity measurements during a complete cardiac cycle.

**Patentansprüche**

1. Verfahren (20) zum Bestimmen eines Maßes der Verengung (15) in einem Gefäß (10), das ein Fluidmedium (11) befördert, wobei das Verfahren die folgenden Schritte umfasst:

a) Nehmen (21) einer Folge von ersten Druckmessungen $P_1$ und einer Folge von entsprechenden ersten Geschwindigkeitsmessungen $U_1$ an einem ersten Ort (5) innerhalb des Gefäßes (10), wobei der erste Ort (5) auf einer ersten Seite eines Zielgebietes (16) liegt;

b) Nehmen (22) einer Folge von zweiten Druckmessungen $P_2$ und einer Folge von entsprechenden zweiten Geschwindigkeitsmessungen $U_2$ an einem zweiten Ort (6) innerhalb des Gefäßes (10), wobei der zweite Ort (6) auf einer zweiten Seite des Zielgebietes (16) liegt;

c) für jeden Ort Bestimmen (23) der Wellengeschwindigkeit c in dem Fluidmedium (11) als eine Funktion des Quadrates einer Änderung des Drucks dP geteilt durch das Quadrat der entsprechenden Änderung der Geschwindigkeit dU;

d) für den ersten Ort (5) Bestimmen (24) einer Vorwärtsdruckänderung $dP_1+$ als eine Funktion der Summe der Änderung des Drucks $dP_1$ und der Änderung der Geschwindigkeit $dU_1$;

e) für den zweiten Ort (6) Bestimmen (25) einer Vorwärtsdruckänderung $dP_2+$ als eine Funktion der Summe der Änderung des Drucks $dP_2$ und der Änderung der Geschwindigkeit $dU_2$;

f) Bestimmen (28) einer getrennten Vorwärtsflussreserve, die den Druckabfall über dem Zielgebiet (16) als eine Funktion des Verhältnisses von $dP_2+ / dP_1+$ angibt.

2. Verfahren gemäß Anspruch 1, bei dem die erste Seite des Zielgebietes (16) stromaufwärts des Zielgebiets und die zweite Seite stromabwärts des Zielgebiets liegen.

3. Verfahren gemäß Anspruch 1, bei dem der Schritt c) ein Bestimmen (23) der Wellengeschwindigkeit c an jedem Ort gemäß der Gleichung c = (1 / $\rho$) $\sqrt{(\sum dP^2 / \sum dU^2)}$ umfasst, wobei $\rho$ die spezifische Dichte des Fluidmediums (11) in dem Gefäß (10) ist.

4. Verfahren gemäß Anspruch 1, bei dem die Schritte d) und e) ein Bestimmen (24, 25) der Vorwärtsdruckänderungen $dP_1+$ und $dP_2+$ gemäß den Gleichungen: $dP_1+ = (dP_1 + pcdU_1) / 2$ und $dP_2+ = (dP_2 + pcdU_2) / 2$ umfassen.

5. Verfahren gemäß Anspruch 1, bei dem der Schritt f) umfasst: Integrieren (26, 27) oder Summieren von mehreren Werten von $dP_1+$ und $dP_2+$, um Vorwärtsdruckwerte $P_1+$ und $P_2+$ zu erhalten, und Bestimmen (28) der getrennten Vorwärtsflussreserve als eine Funktion des Verhältnisses $P_2+ / P_1+$.

6. Verfahren gemäß Anspruch 1, das auf ein Gefäß (10) angewandt wird, bei dem es eine Quelle eines schwankenden Drucks auf jeder Seite des Zielgebietes (16) gibt.

7. Verfahren gemäß Anspruch 6, das auf ein Gefäß (10) im menschlichen oder tierischen kardialen Blutkreislaufsystem angewendet wird.

8. Verfahren gemäß Anspruch 7, bei dem die Folge von ersten und zweiten Druckmessungen und die Folge von ersten und zweiten Geschwindigkeitsmessungen über mindestens einen vollständigen Herzzyklus genommen werden (21, 22).

9. Verfahren gemäß Anspruch 1, bei dem entsprechende Druck- und Geschwindigkeitsmessungen gleichzeitig genommen werden.

10. Vorrichtung zum Bestimmen eines Maßes der Verengung (15) in einem Gefäß (10), das ein Fluidmedium (11) befördert, wobei die Vorrichtung umfasst:

einen Drucksensor (30) und einen Geschwindigkeitssensor (32) zum Nehmen (21, 22) einer Folge von Druck- und Geschwindigkeitsmessungen in dem Gefäß (10) an mindestens einem ersten Ort (5) stromaufwärts eines Zielgebiets (16) und einem zweiten Ort (6) stromabwärts des Zielgebiets (16);
ein Verarbeitungsmodul (35, 36), das angepasst ist, um:

eine Folge von ersten Druckmessungen $P_1$ und eine Folge von entsprechenden ersten Geschwindigkeitsmessungen $U_1$ zu empfangen, die an dem ersten Ort (5) innerhalb des Gefäßes (10) genommen werden;
eine Folge von zweiten Druckmessungen $P_2$ und eine Folge von entsprechenden zweiten Geschwindigkeitsmessungen $U_2$ zu empfangen, die an dem zweiten Ort (6) innerhalb des Gefäßes (10) genommen werden;
für jeden Ort die Wellengeschwindigkeit c in dem Fluidmedium (11) als eine Funktion des Quadrates einer Änderung des Drucks dP geteilt durch das Quadrat der entsprechenden Änderung der Geschwindigkeit dU zu bestimmen (23);
für den ersten Ort (5) eine Vorwärtsdruckänderung $dP_1+$ als eine Funktion der Summe der Änderung des Drucks $dP_1$ und der Änderung der Geschwindigkeit $dU_1$ zu bestimmen (24); und
für den zweiten Ort (6) eine Vorwärtsdruckänderung $dP_2+$ als eine Funktion der Summe der Änderung des Drucks $dP_2$ und der Änderung der Geschwindigkeit $dU_2$ zu bestimmen (25); und
eine getrennte Vorwärtsflussreserve zu bestimmen (28), die den Druckabfall über dem Zielgebiet (16) als eine Funktion des Verhältnisses von $dP_2+ / dP_1+$ angibt.

11. Vorrichtung gemäß Anspruch 10, bei der das Verarbeitungsmodul (36) ferner angepasst ist, um die Wellengeschwindigkeit c an jedem Ort gemäß der Gleichung c = (1 / $\rho$) $\sqrt{(\sum dP^2 / \sum dU^2)}$ zu bestimmen (23), wobei $\rho$ die spezifische Dichte des Fluidmediums (11) in dem Gefäß (10) ist.

12. Vorrichtung gemäß Anspruch 10, bei der das Verarbeitungsmodul (36) ferner angepasst ist, um die Vorwärtsdruckänderungen $dP_1+$ und $dP_2+$ gemäß den Gleichungen: $dP_1+ = (dP_1 + pcdU_1) / 2$ und $dP_2+ = (dP_2 + pcdU_2) / 2$ zu bestimmen (24, 25).

13. Vorrichtung gemäß Anspruch 10, bei der das Verarbeitungsmodul (36) ferner angepasst ist, um mehrere Werte von $dP_1+$ und $dP_2+$ zu integrieren (26, 27) oder aufzusummieren, um Vorwärtsdruckwerte $P_1+$ und $P_2+$ zu erhalten und die getrennte Vorwärtsflussreserve als eine Funktion des Verhältnisses $P_2+ / P_1+$ zu bestimmen (28).

**14.** Vorrichtung gemäß Anspruch 10, die ferner Mittel zum Überwachen eines Herzrhythmus und zum Steuern des Drucksensors und des Geschwindigkeitssensors umfasst, um die Folge von Druckmessungen und die Folge von Geschwindigkeitsmessungen während eines vollständigen Herzzyklus zu sammeln.

**Revendications**

**1.** Procédé (20) de détermination d'une mesure de constriction (15) dans un vaisseau (10) transportant un milieu fluidique (11), le procédé comprenant les étapes :

a) de prise (21) d'une succession de premières mesures de pression $P_1$ et d'une succession de premières mesures de vitesse $U_1$ correspondantes à un premier emplacement (5) dans le vaisseau (10), le premier emplacement (5) étant d'un premier côté d'une région cible (16) ;
b) de prise (22) d'une succession de deuxièmes mesures de pression $P_2$ et d'une succession de deuxièmes mesures de vitesse $U_2$ correspondantes à un deuxième emplacement (6) dans le vaisseau (10), le deuxième emplacement (6) étant d'un deuxième côté de la région cible (16) ;
c) pour chaque emplacement, de détermination (23) d'une vitesse d'onde c dans le milieu fluidique (11) en fonction du carré d'une variation de pression dP divisé par le carré de la variation de vitesse dU correspondante ;
d) pour le premier emplacement (5), de détermination (24) d'une variation de pression en avant $dP_1+$ en fonction de la somme de la variation de pression $dP_1$ et de la variation de vitesse $dU_1$ ;
e) pour le deuxième emplacement (6), de détermination (25) d'une variation de pression en avant $dP_2+$ en fonction de la somme de la variation de pression $dP_2$ et de la variation de vitesse $dU_2$ ;
f) de détermination (28) d'une réserve d'écoulement séparée en avant indicative de la chute de pression à travers la région cible (16) en fonction du rapport $dP_2+/dP_1+$.

**2.** Procédé selon la revendication 1, dans lequel le premier côté de la région cible (16) est en amont de la région cible et le deuxième côté est en aval de la région cible.

**3.** Procédé selon la revendication 1, dans lequel l'étape c) comprend la détermination (23) de la vitesse d'onde c à chaque emplacement par l'équation $c = (1/p) \sqrt{(\sum dP^2/\sum dU^2)}$, où p est la densité spécifique du milieu fluidique (11) dans le vaisseau (10).

**4.** Procédé selon la revendication 1, dans lequel les étapes d) et e) comprennent la détermination (24, 25) desdites variations de pression en avant $dP_1+$ et $dP_2+$ par les équations : $dP_1+ = (dP_1 + pcdU_1)/2$ et $dP_2+ = (dP_2 + pcdU_2)/2$.

**5.** Procédé selon la revendication 1, dans lequel l'étape f) comprend l'intégration (26, 27) ou la sommation de multiples valeurs de $dP_1+$ et $dP_2+$ de manière à obtenir les valeurs de pression en avant $P_1+$ et $P_2+$ et la détermination (28) de la réserve d'écoulement séparée en avant en fonction du rapport $P_2+/P_1+$.

**6.** Procédé selon la revendication 1 appliqué à un vaisseau (10), dans lequel il existe une source de pression fluctuante de chaque côté de la région cible (16).

**7.** Procédé selon la revendication 6 appliqué à un vaisseau (10) dans un système circulatoire cardiaque d'un être humain ou d'un animal.

**8.** Procédé selon la revendication 7, dans lequel la succession de premières et deuxièmes mesures de pression et la succession de premières et deuxièmes mesures de vitesse sont prises (21, 22) pendant au moins un cycle cardiaque complet.

**9.** Procédé selon la revendication 1, dans lequel les mesures de pression et de vitesse correspondantes sont prises simultanément.

**10.** Appareil pour déterminer une mesure de constriction (15) dans un vaisseau (10) transportant un milieu fluidique (11), l'appareil comprenant :

un capteur de pression (30) et un capteur de vitesse (32) pour prendre (21, 22) une succession de mesures de pression et de vitesse dans le vaisseau (10) à au moins un premier emplacement (5) en amont d'une région cible (16) et à un deuxième emplacement (6) en aval de la région cible (16) ;

un module de traitement (35, 36) conçu pour :

recevoir une succession de premières mesures de pression $P_1$ et une succession de premières mesures de vitesse $U_1$ correspondantes prises au premier emplacement (5) dans le vaisseau (10) ;

recevoir une succession de deuxièmes mesures de pression $P_2$ et une succession de deuxièmes mesures de vitesse $U_2$ correspondantes prises au deuxième emplacement (6) dans le vaisseau (10) ;

pour chaque emplacement, déterminer une vitesse d'onde c (23) dans le milieu fluidique (11) en fonction du carré d'une variation de pression dP divisé par le carré de la variation de vitesse dU correspondante ;

pour le premier emplacement (5), déterminer (24) une variation de pression en avant $dP_1+$ en fonction de la somme de la variation de pression $dP_1$ et de la variation de vitesse $dU_1$ ; et

pour le deuxième emplacement (6), déterminer (25) une variation de pression en avant $dP_2+$ en fonction de la somme de la variation de pression $dP_2$ et de la variation de vitesse $dU_2$ ; et

déterminer (28) une réserve d'écoulement séparée en avant indicative de la chute de pression à travers la région cible (16) en fonction du rapport $dP_2+/dP_1+$.

11. Appareil selon la revendication 10, dans lequel le module de traitement (36) est en outre conçu pour déterminer (23) la vitesse d'onde c à chaque emplacement par l'équation $c = (1/\rho) \sqrt{(\sum dP^2/\sum dU^2)}$, où p est la densité spécifique du milieu fluidique (11) dans le vaisseau (10).

12. Appareil selon la revendication 10, dans lequel le module de traitement (36) est en outre conçu pour déterminer (24, 25) lesdites variations de pression en avant $dP_1+$ et $dP_2+$ par les équations $dP_1+ = (dP_1 + pcdU_1)/2$ et $dP_2+ = (dP_2 + pcdU_2)/2$.

13. Appareil selon la revendication 10, dans lequel le module de traitement (36) est en outre conçu pour intégrer (26, 27) ou sommer de multiples valeurs de $dP_1+$ et $dP_2+$ pour obtenir les valeurs de pression en avant $P_1+$ et $P_2+$ et pour déterminer (28) la réserve d'écoulement séparée en avant en fonction du rapport $P_2+/P_1+$.

14. Appareil selon la revendication 10, comprenant en outre des moyens pour surveiller un rythme cardiaque et pour commander ledit capteur de pression et ledit capteur de vitesse pour collecter ladite succession de mesures de pression et ladite succession de mesures de vitesse pendant un cycle cardiaque complet.

Figure. 1

20

```
┌─────────────────────────────────┐
│ MEASURE PRESSURES P1 AND        │──21
│ VELOCITIES U1 AT FIRST LOCATION │
└─────────────────────────────────┘
              │
              ▼
┌─────────────────────────────────┐
│ MEASURE PRESSURES P2 AND        │──22
│ VELOCITIES U2 AT SECOND LOCATION│
└─────────────────────────────────┘
              │
              ▼
┌─────────────────────────────────┐
│ DETERMINE WAVE VELOCITY         │
│ AT EACH LOCATION                │──23
│                                 │
│                                 │
└─────────────────────────────────┘
```

$$C = \frac{1}{\rho} \sqrt{\frac{\Sigma dP^2}{\Sigma dU^2}}$$

DETERMINE FORWARD PRESSURE CHANGE AT FIRST LOCATION: —24

$$dP_1+ = \frac{dP_1 + \rho c dU_1}{2}$$

DETERMINE FORWARD PRESSURE CHANGE AT SECOND LOCATION: —25

$$dP_2+ = \frac{dP_2 + \rho c dU_2}{2}$$

INTEGRATE / SUM OVER ALL $dP_1 + \longrightarrow P_1 +$ —26

INTEGRATE / SUM OVER ALL $dP_2 + \longrightarrow P_2 +$ —27

DETERMINE FRACTIONAL FLOW RESERVE AS A FUNCTION OF RATIO $P_2+ / P_1+$ —28

Figure 2

Figure. 3

Figure 4

Figure 5

Fractional Flow Reserve

Percentage stenosis

Figure 6

Pressure above diastolic (mmHg)

Forward-travelling pressure

Backward-travelling pressure

Total pressure

Time (ms)

EP 2 544 585 B1

**REFERENCES CITED IN THE DESCRIPTION**

**Non-patent literature cited in the description**

- **N. HADJILOIZOU et al.** Differences in cardiac microcirculatory wave patterns between the proximal left mainstem and proximal right coronary artery. *Am. J. Physiol.: Heart Circ. Physiol.,* 18 July 2008, vol. 295, H1198-H1205
- **J E DAVIES et al.** Evidence of a dominant backward-propagating "suction" wave responsible for diastolic coronary filling in humans, attenuated in left ventricular hypertrophy. *Circulation,* 11 April 2006, vol. 113 (14), 1768-78
- **J E DAVIES et al.** Use of simultaneous pressure and velocity measurements to estimate arterial wave speed at a single site in humans. *Am J Physiol Heart Circ Physiol,* February 2006, vol. 290 (2), H878-H885
- **DAWKINS KD ; GERSHLICK T, DE BM et al.** Percutaneous coronary intervention: recommendations for good practice and training. *Heart,* December 2005, vol. 91 (6), vi1-27
- **BLOWS LJ ; REDWOOD SR.** The pressure wire in practice. *Heart,* April 2007, vol. 93 (4), 419-22
- **PIJLS NH ; VAN SON JA ; KIRKEEIDE RL, DE BB ; GOULD KL.** Experimental basis of determining maximum coronary, myocardial, and collateral blood flow by pressure measurements for assessing functional stenosis severity before and after percutaneous transluminal coronary angioplasty. *Circulation,* April 1993, vol. 87 (4), 1354-67
- **PIJLS NH, DE BB ; PEELS K et al.** Measurement of fractional flow reserve to assess the functional severity of coronary-artery stenoses. *N Engl J Med,* 27 June 1996, vol. 334 (26), 1703-8
- **PIJLS NH ; KLAUSS V ; SIEBERT U et al.** Coronary pressure measurement after stenting predicts adverse events at follow-up: a multicenter registry. *Circulation,* 25 June 2002, vol. 105 (25), 2950-4
- **SPAAN JA ; BREULS NP ; LAIRD JD.** Diastolic-systolic coronary flow differences are caused by intramyocardial pump action in the anesthetized dog. *Circ Res,* September 1981, vol. 49 (3), 584-93
- **GREGG DE ; SABISTON DC.** Effect of cardiac contraction on coronary blood flow. *Circulation,* January 1957, vol. 15 (1), 14-20
- **DAVIES JE ; WHINNETT ZI ; FRANCIS DP et al.** Evidence of a dominant backward-propagating "suction" wave responsible for diastolic coronary filling in humans, attenuated in left ventricular hypertrophy. *Circulation,* 11 April 2006, vol. 113 (14), 1768-78

- **KRAMS R ; SIPKEMA P ; WESTERHOF N.** Varying elastance concept may explain coronary systolic flow impediment. *Am J Physiol,* November 1989, vol. 257, H1471-H1479
- **HIRAMATSU O ; GOTO M ; YADA T et al.** In vivo observations of the intramural arterioles and venules in beating canine hearts. *J Physiol,* 01 June 1998, vol. 509, 619-28
- **CHILIAN WM ; MARCUS ML.** Phasic coronary blood flow velocity in intramural and epicardial coronary arteries. *Circ Res,* June 1982, vol. 50 (6), 775-81
- **SIEBES M ; CHAMULEAU SA ; MEUWISSEN M ; PIEK JJ ; SPAAN JA.** Influence of hemodynamic conditions on fractional flow reserve: parametric analysis of underlying model. *Am J Physiol Heart Circ Physiol,* October 2002, vol. 283 (4), H1462-H1470
- **SPAAN JA.** Coronary flow is not that simple!. *Heart.,* May 2009, vol. 95 (9), 761-2
- **DAVIES JE ; HADJILOIZOU N ; FRANCIS DP ; HUGHES AD ; PARKER KH ; MAYET J.** The role of the coronary microcirculation in determining blood flow. *Artery Research,* 2006, vol. 1 (S1), S31-S32
- **KIM RJ ; WU E ; RAFAEL A et al.** The use of contrast-enhanced magnetic resonance imaging to identify reversible myocardial dysfunction. *N Engl J Med,* 16 November 2000, vol. 343 (20), 1445-53
- **PERERA D ; BIGGART S ; POSTEMA P et al.** Right atrial pressure: can it be ignored when calculating fractional flow reserve and collateral flow index?. *J Am Coll Cardiol,* 16 November 2004, vol. 44 (10), 2089-91
- **DAVIES JE ; WHINNETT ZI ; FRANCIS DP et al.** Use of simultaneous pressure and velocity measurements to estimate arterial wave speed at a single site in humans. *Am J Physiol Heart Circ Physiol,* February 2006, vol. 290 (2), H878-H885
- **PARKER KH ; JONES CJ ; DAWSON JR ; GIBSON DG.** What stops the flow of blood from the heart?. *Heart Vessels,* 1988, vol. 4 (4), 241-5
- **DAVIES JE ; PARKER KH ; FRANCIS DP ; HUGHES AD ; MAYET J.** What is the role of the aorta in directing coronary blood flow?. *Heart,* December 2008, vol. 94 (12), 1545-7

- **HADJILOIZOU N ; DAVIES JE ; MALIK IS et al.** Differences in cardiac microcirculatory wave patterns between the proximal left mainstem and proximal right coronary artery. *Am J Physiol Heart Circ Physiol,* September 2008, vol. 295 (3), H1198-H1205